# EUROPÄISCHE PATENTSCHRIFT

(11) **EP 1 906 890 B1**
(45) Veröffentlichungstag und Bekanntmachung des Hinweises auf die Patenterteilung: **21.10.2009**
(21) Anmeldenummer: 07722929.2
(22) Anmeldetag: 26.02.2007
(51) Int. Cl.: A61F 5/01

(54) **SPRUNGGELENKBANDAGE**
ANKLE JOINT BANDAGE
BANDAGE POUR L'ARTICULATION DE LA CHEVILLE

(30) Priorität: 27.02.2006 DE 202006003245 U
(43) Veröffentlichungstag der Anmeldung: 09.04.2008
(73) Patentinhaber: Thuasne Deutschland GmbH, 35099 Burgwald (DE)
(72) Erfinder: BRZANK, Klaus-Dieter, 58540 Meinerzhagen (DE)
(74) Vertreter: Buchhold, Jürgen
(86) Internationale Anmeldenummer: PCT/EP2007/001617
(87) Internationale Veröffentlichungsnummer: WO 2007/098898

(56) Entgegenhaltungen:
- EP-A1- 0 485 943
- WO-A-01/85078
- WO-A-20/04098467
- DE-A1- 3 838 582
- DE-A1- 10 305 277
- DE-A1-102004 019 007
- DE-C2- 4 104 930
- DE-C2- 4 318 791
- DE-U1- 9 115 983
- DE-U1- 20 005 742
- US-A- 4 729 370

## Beschreibung

Die vorliegende Erfindung betrifft eine Sprunggelenkbandage mit einem anatomisch geformten socken- oder strumpfartigen Grundkörper, welcher sich zumindest zwischen einem Unterschenkelabschnitt und einem Fußabschnitt erstreckt und aus einem dehnbaren Textilmaterial ausgebildet ist. Insbesondere betrifft die Erfindung eine Sprunggelenkbandage, welche in Abhängigkeit der Anwendung bzw. der medizinischen Indikation weitere Merkmale aufweist, welche beispielsweise Pelottenkörper oder als Pronationszügel wirkende Versteifungen in der Bandage betreffen.

Das Sprunggelenk zählt zu einem wesentlichen Bestandteil des Stütz- und Bewegungsapparates des menschlichen Körpers. Es ist insbesondere bei sportlichen Aktivitäten hohen Stoß- und Druckbelastungen ausgesetzt. Bei Überbelastungen kann es zu Traumatisierungen der Weichteile kommen, beispielsweise zu Supinationstraumen in Folge starker Überdehnung der Bänder. Aber auch ein Umknicken des Fußes über den lateralen Fußrand kann zu Verletzungen im Sprunggelenk führen. Dabei treten Schäden unterschiedlicher Ausprägung auf. Voraussetzung für die Funktionsfähigkeit des Sprunggelenks ist daher ein funktionierender Kapsel-Band-Apparat.

Grundsätzlich wird zwischen dem oberen und dem unteren Sprunggelenk unterschieden. Das obere Sprunggelenk erlaubt als Scharniergelenk nur Streck- und Beugebewegungen. Das als die Tibia bezeichnete Schienbein und das als die Fibula bezeichnete Wadenbein umfassen zangenförmig die Gelenkrolle des Sprungbeins (Trochlea tali) und bilden die Malleolengabel. Das innere und äußere Kollateralband bilden eine zusätzliche Stabilität, wobei die Bänder in ihrer Verlaufsstruktur gefächert sind, so dass in Abhängigkeit von der Gelenkstellung jeweils ein Teil angespannt ist. Das untere Sprunggelenk lässt Drehbewegungen um eine von lateral hinten nach medial vorne oben gerichtete Schrägachse zu, so dass die Pronation und die Supination des Fußes ermöglicht werden. Als Pronation bezeichnet man eine nach außen gerichtete Drehung, wohingegen die Supination eine Drehung des Fußes nach innen beschreibt.

In der Sportmedizin gilt das Sprunggelenk nach dem Kniegelenk als der am zweithäufigsten betroffene Verletzungsbereich. Dabei sind zur Prävention von Verletzungen, zum Schutz bzw. zur Stabilisierung und zum Erhalt der Sportivität sowie für Nachbehandlungen Bandagen anwendbar, welche insbesondere zur Behandlung von Fußgelenkserkrankungen verschiedene Ausführungen aufweisen. So gibt es schlauch- bzw. strumpfförmige Bandagen, die den medialen und lateralen Malleolus der Unterschenkelknochen mittels einer Silikonpelotte umgeben, wodurch einmal die Durchblutung gefördert sowie Stauungen vermieden werden und zum Zweiten eine Stützung des oberen Sprunggelenks erzielt wird.

Andere Ausführungen von Sprunggelenkbandagen sind mit so genannten Pronationszügeln ausgebildet, welche vorzugsweise außenseitig am Fuß angebracht sind, um eine Supination des Fußes zu vermeiden und um eine Heilung eines verletzten Kapsel-Band-Apparates zu gewährleisten. Weiterhin können Fußgelenkbandagen an unterschiedlichen Regionen des Fußes Pelottenkörper aufweisen, welche zumeist als feste Verstärkungen im Textilgewebe des Bandagenkörpers eingearbeitet sind und auf die entsprechende Fußregion Druck ausüben, einen Massageeffekt hervorrufen oder man nutzt ihre stabilisierende Wirkung. Zumeist sind diese Pelotten als Silikoneinlagen ausgeführt, welche als Formkörper im textilen Grundkörper der Bandage eingearbeitet und in ihrer Kontur sowie in ihrer Dicke der Anatomie der jeweiligen Fußregion angepasst sind.

Die Erfindung geht von dem Dokument DE-C2-41 04 930 als nächstliegendem Stand der Technik aus.

In DE-C2-41 04 930 ist eine schlauchförmige Bandage beschrieben, die das Sprunggelenk begrenzt seitlich abstützt und stabilisierend auf den Kapsel-Band-Apparat einwirkt und zwar bei gleichzeitiger Abpolsterung des kritischen Bereichs der Bisgaard'schen Kulisse, um eine gleichmäßige Druckverteilung zu erzielen, wobei eine durch die Bewegung hervorgerufene intermittierende Kompression auf die Gelenkweichteile um den medialen und lateralen Malleolus und eine tiefe Querfriktion auf den Ansatz der Achilles-Sehne ausgeübt wird. Als Abpolsterung werden dabei besonders ausgebildete Pelotten verwendet. Die Pelotten sind als Formkörper mit einer seitlichen, abgerundeten Aussparung zur Aufnahme des Knöchelvorsprungs ausgebildet der aus einem weichen oder weich-elastischen Material besteht, wobei in den Formkörper ein stabförmiger Friktionskern aus einem harten inkompressiblen Material angeordnet ist.
Eine Anwendung eines Pronationszügels ist in DE-C2-43 18 791 offenbart. Die hierin beschriebene Sprunggelenksbandage weist einen vom Fersenbereich über das Sprunggelenk gerade hoch reichenden Pronationszügel aus reiß- und zugfestem Material sowie auf der Innenseite einer in gleicher Richtung verlaufende Versteifungseinlage auf. Um den Pronationszügel und die Versteifungseinlage festzuhalten, ist weiterhin ein elastischer Gurt vorgesehen, der im Fersenbereich des Fußabschnittes angebracht ist und von dort aus über den Spann des Fußes und um das Sprunggelenk herum geschlungen ist und mit seinem Ende nach Umschlingung des Sprunggelenks am Gurt im Bereich seiner Umschlingung des Spannes in diesem Bereich festgelegt ist.

DE-U1-200 05 742 offenbart eine Sprunggelenkbandage mit einem sich mindestens über den Mittelfuß und das Sprunggelenk erstreckenden Strumpf und einem daran unterhalb der Fußsohle ansetzenden und in Richtung der Fußaußenseite verlaufenden Befestigungsband zur Umschlingung des Unterschenkels, wobei das Befestigungsband in seinem Umschlingungsweg über die Fußaußenseite und über den Spann des Fußes als Anfangsteil aus elastischem Bandmaterial und anschließend in mindestens vollständiger Umschlingung des Unterschenkels als Endteil in Ausführung aus unelastischem Bandmaterial verläuft und an seinem Ende mit einem Klettverschlussstück versehen ist, welches an einem entsprechenden Klettverschlussgegenstück an der Sprunggelenksbandage festlegbar ist.

Eine ähnliche ein Bandmaterial umfassende Bandage ist aus DE-A1-38 38 582 bekannt, welche eine gelenkbedeckende, elastische Knöchelsocke offenbart, die aus einer schlauchförmigen Strickware ausgebildet ist, wobei die Form und die Größe dem zu bandagierenden Sprunggelenk anatomisch angepasst ist, und wobei diese einen in die Knöchelsocke integrierten U-förmigen Steigbügeleinsatz nach Art einer antagonistischen Schlinge umfasst, der von einem um den Knöchel gelegten Anker spiralförmig umschlungen wird, wobei der Anker als unelastisches Band mit Abstand seiner Windungen geführt ist und an dem äußeren Schenkel des Steigbügeleinsatzes beginnt und endet.

Hinsichtlich einer Silikon-Außenpelotte offenbart die DE-A1-103 05 277 eine Fußbandage, welche insbesondere im lateralen Bereich der Achillessehne einen über einen Unterschenkelbereich, den anschließenden Fersen-Gelenk-Bereich und den Mittelfußbereich verlaufenden strumpfförmigen Schlauch aus elastischem dehnbaren Textilmaterial umfasst, wobei der Schlauch im Unterschenkelbereich zu beiden Seiten der Achillessehne mit einer Pelotte aus elastischem Material bestückt ist. Die an der Innenseite des Unterschenkelbereichs des Schlauches liegende Pelotte ist mit einem in den Fußgewölbebereich des Schlauches verlaufenden inneren Verlängerungsstück versehen. Diese Silikonpelotten und deren Verlängerungsstücke sind im Querschnitt etwa linsenförmig ausgebildet, wobei die innenseitigen Bereiche Noppen aufweisen, um einen verstärkten Massageeffekt zu erzielen.

WO 2004/098467 A1 offenbart eine Knöchelbandage, die sich entlang des lateralen und medialen Malleolus eines verletzten Knöchels und Unterschenkels erstreckt, um den Knöchel während der Heilung zu unterstützen. Der laterale Malleolus und der Unterschenkel werden von zwei starren Schalenkörpern urnschlossen, die nach unten hin in der Breite und der Wandstärke abnehmen, um in einen Schuh zu passen. Der Randbereich der beiden Schalenkörper ist mit einem flexiblen Material umgeben, damit das Bein und der Knöchel von den Stosskanten des starren Stützkörpers geschützt sind. Die beiden Schalenkörper werden durch Bänder mit Klettverschlüssen miteinander verbunden. Die beiderseitigen Stützkörper sind starr ausgebildet und nicht aus einem dehnbaren Textilmaterial gefertigt.

DE 91 15 983 U1 beschreibt eine Knöchelstütze, mit einem elastischen, vorne offenen, den proximalen Teil des Fußes, den Knöchel und einen Teil des Unterschenkels umfassenden Strumpf, mit einem im Sohlenbereich vorgesehenen Pronationskeil. Zur Polsterung des Knöchelbereiches sind an dem Strumpf Silikonpolster vorgesehen. Die als elastische Knöchelstütze bei dieser Vorrichtung eingesetzten Silikonpolster entfallen bei der neuen Erfindung. Somit entsteht mehr Platz im Schuh, wodurch der Tragekomfort erhöht wird.

Die Knöchelstütze in der US 4,729,370 B besitzt eine dehnbare Zwischenschicht, die sich wie ein Strumpf, der sich bis zu einem gewissen Punkt über dem Knöchel erstreckt, an den Fuß des Trägers anpasst. Ein nicht dehnbarer seitlicher Riemen ist an der Zwischenschicht, unterhalb des Knöchels, befestigt und erstreckt sich bis an das obere Ende der Zwischenschicht, an dem es unelastisch befestigt wird. Der seitliche Riemen ist zusätzlich mit einem ebenfalls nicht dehnbaren medialen Riemen verbunden. Nach Anbringen dieser Knöchelstütze ist die Bewegung des Knöchels stark eingeschränkt. Eine Pelotte oder eine andere Polsterung um den Knöchel herum ist bei dieser Stütze nicht vorgesehen.

WO 01/85078 A1 beschreibt eine Sprunggelenk-Bandage, die aus einem länglichen Band besteht, wobei das längliche Band um die Ferse, über die laterale Seite des Sprunggelenks, über den Fußrücken nach medial plantar und über die Fußsohle nach lateral plantar geführt wird, wobei die erste und die zweite Querkante des Bandes am Band selbst befestigt sind. Am länglichen Band setzen zwei Zügel an, ein erster ausgehend von der lateralen Seite am Vorfuß und ein zweiter von der lateralen Seite am Sprunggelenk, wobei der zweite Zügel zirkulär um das Sprunggelenk geschlossen wird und der erste Zügel am Vorfuß über den Fußrücken zur medialen Seite des Sprunggelenks geführt und zirkulär um das Sprunggelenk geschlossen wird. Diese Sprunggelenk-Bandage weist ebenfalls keine ausgebildete Pelotte auf.

EP 0 485 943 A1 offenbart eine viskoelastische Profileinlage, mit einer großflächigen Ausbildung, die im Tragzustand neben dem Knöchelfurchbereich auch noch den Knöchelbereich selbst und den Achillessehnenbereich und den Fersenbereich und den Fußsohlenbereich des Fußes partiell abdeckt. Für die Pelotte dieser Sprunggelenk-Bandage wird ein Additions-Silikon verwendet. Das eingesetzte Silikon engt den Platz im Schuh ein, wodurch der Tragekomfort geschmälert wird.

Die Orthese in der DE 10 2004 019 007 A1 besitzt ein Federelement, dessen Federkraft bei angelegter Orthese einem Strecken des Fußes entgegenwirkt, wobei die Orthese einen einstückigen Grundkörper besitzt, der strumpfartig geformt ist. Der hier beschriebene Grundkörper ist komplett aus Silikon gefertigt. Durch das gummiartige Gewebe kann es sehr schnell zu einem Hitzestau am Fuß kommen, was zu erhöhter unangenehmer Schweißbildung führen kann.

Bei den bekannten Sprunggelenkbandagen tritt das Problem auf, dass diese häufig vollständig aus elastischem Material hergestellt sind, wobei ihr stabilisierender Einfluss vor allem auf den priozeptiven Faktor begrenzt bleibt, welcher die Eigenwahrnehmung des Körpers betrifft. Die Bandagen sind zudem häufig mehrteilig ausgebildet und erfordern ein aufwendiges Anlegen an das Sprunggelenk, was meist sehr viel Übung und oft entsprechende Fachkenntnisse beim Patienten erfordert.

Der Tragekomfort der bekannten Bandagen wird häufig dadurch eingeschränkt, dass die physiologisch wirksamen Ausbildungen an den Bandagen wie die Pronationszügel, die Pelotten über den Malleolen sowie Silikon-Außenpelotten relativ viel Platz einnehmen, was insbesondere beim Anlegen eines Schuhwerks Schmerzen verursachen und eine Heilung des Kapsel-Band-Apparates negativ beeinflussen kann. Weiterhin bieten die bekannten Sprunggelenkbandagen keine sinnvolle Kombination mehrerer die Heilung des Kapsel-Band-Apparates unterstützenden Vorrichtungen, so dass maximal eine Teilwirkung auf eine begrenzte Region des Fußes erzielbar ist.

Ziel der Erfindung ist es daher, die Nachteile des Standes der Technik zu überwinden und eine Sprunggelenkbandage zu schaffen, die eine einfache Anwendung ermöglicht und eine stets optimale Unterstützung des Kapsel-Band-Apparates des Fußes im Bereich des Sprunggelenks ermöglicht.

Diese Aufgabe wird ausgehend von einer Sprunggelenkbandage gemäß dem Oberbegriff von Anspruch 1 in Verbindung mit den kennzeichnenden Merkmalen gelöst. Vorteilhafte Weiterbildungen der Erfindung sind in den abhängigen Ansprüchen angegeben.

Bei einer Sprunggelenkbandage mit einem anatomisch geformten socken- oder strumpfartigen Grundkörper, welcher sich zumindest zwischen einem Unterschenkelabschnitt und einem Fußabschnitt erstreckt und aus einem dehnbaren Textilmaterial ausgebildet ist, wobei die Bandage im Bereich über wenigstens einer Malleolenregion eine Pelotte umfasst, sieht die Erfindung vor, dass die zumindest eine Pelotte als 3D-Gestrick im Grundkörper ausgeformt ist.
Die Erfindung geht dabei von dem Gedanken aus, dass über der Malleolenregion eine Pelotte ausgebildet ist, welche nicht durch einen Formkörper aus einem harten Material besteht, sondern direkt im Textilmaterial ausgeformt ist. Ein wesentlicher Vorteil ist darin zu sehen, dass eine beispielsweise aus dem Silikonmaterial bestehende Pelotte entfallen kann, welche zur Druckverteilung und zur Entlastung der Knöchelvorsprünge dient. Somit entsteht mehr Platz im Schuh und ein Engegefühl entfällt, womit sich der Tragekomfort erhöht.

Dabei ist es zweckmäßig, wenn das dehnbare Textilmaterial des Grundkörpers im Übergang in das die Pelotte bildende 3D-Gestrick eine Texturänderung aufweist, welche eine lateral nach außen gerichtete Dehnbarkeit bewirkt, die höher ist als die Dehnbarkeit des Textilmaterials des Grundkörpers. Durch diese dreidimensionale Ausformung des Gestricks über der Malleolenregion reduziert sich die Kompression, wobei das 3D-Gestrick die Funktion der Pelotte selbst übernimmt. Das ermöglicht es zudem, die Dicke der perimalleolären Silikonpelotte zu reduzieren.

Im gedehnten Zustand des 3D-Gestricks bildet diese kugelkappenförmige Ausbildungen in lateraler Richtung im Bereich der Malleolenregion, welche die Malleolen selbst einformen. Das dehnbare Textilmaterial weist dabei einen gummiartigen Gewebeanteil auf, wobei die Texturänderung des dehnbaren Textilmaterials im Übergang vom Grundkörper in das 3D-Gestrick eine Reduzierung des gummiartigen Gewebeanteils umfasst.

Das dehnbare Textilmaterial, welches ebenfalls den Grundkörper der Sprunggelenkbandage bildet, geht einteilig durchgehend in das die Pelotte bildende 3D-Gestrick über, wobei das Textilmaterial lediglich eine Texturänderung aufweist. Die Texturänderung betrifft insbesondere einen reduzierten Anteil des gummiartigen Gewebes in der Pelotte, welches für die Elastizität sorgt, und somit die Druckkraft auf den Fuß ausübt. Durch den reduzierten Anteil des gummiartigen Gewebes reduziert sich zugleich die Kompression, d.h. die Flächenpressung auf die Malleolenregion. Im gespannten Zustand erhebt sich somit das Gestrick aus der Ebene des Textilmaterials in einer dritten Dimension hervor, wobei sich keine wesentliche Erhöhung der Flächenpressung ergibt.

Eine weitere vorteilhafte Ausgestaltung der Erfindung sieht vor, dass der gummiartige Gewebeanteil des dehnbaren Textilmaterials eine Vorspannung aufweist, wobei die Vorspannung im Bereich des 3D-Gestrickes geringer ist als im dehnbaren Textilmaterial des Grundkörpers. Insbesondere im Übergang, d.h. im Randbereich des 3D-Gestricks weist der gummiartige Gewebeanteil eine geringere Vorspannung auf, wobei unter der Vorspannung die im ungedehnten Zustand des Textilmaterials herrschende Längsspannung im gummiartigen Gewebeanteil verstanden wird.

Das die Pelotte bildende 3D-Gestrick ist im Wesentlichen kreisförmig ausgebildet, wobei der Durchmesser des kreisförmigen 3D-Gestricks im ungedehnten Zustand etwa 15 mm bis 35 mm, vorzugsweise etwa 20 mm bis 30 mm und besonders bevorzugt etwa 25 mm beträgt. Die Größe der damit entstehenden Pelotte umschließt im Wesentlichen die Malleolenregion, wobei sich der angegebene Durchmesser im Zustand des gedehnten Materials des 3D-Gestrickes um bis zu 50 %, im Einzelfall auch bis zu 100 % vergrößern kann. Je nach Ausführung des 3D-Gestrickes bzw. der geforderten Form der Pelotte kann das 3D-Gestrick im ungedehnten Zustand auch eine elliptische Form annehmen, wobei die Ellipse in ihrer Längsausrichtung der Längsausrichtung des Unterschenkelabschnitts der Sprunggelenkbandage entspricht.

Im nicht angelegten Zustand beträgt die Höhe des Unterschenkelabschnitts vom oberen Rand bis zum unteren Fersenrand etwa 200 - 220 mm, wobei das die Pelotte bildende 3D-Gestrick bezogen auf den Mittelpunkt der Pelotte einen Abstand von etwa 100 - 110 mm vom oberen Unterschenkelabschnitt aufweist. In einem planen, ungedehnten Zustand der Sprunggelenkbandage beträgt deren Breite etwa 90 - 110 mm, wobei das die Pelotte bildende 3D-Gestrick vom vorderfußseitigen Rand im Unterschenkelabschnitt des Grundkörpers bezogen auf den Mittelpunkt der Pelotte einen Abstand von etwa 30 - 40 mm aufweist.

Vorteilhafterweise ist das die Pelotte bildende 3D-Gestrick im dehnbaren Textilmaterial des Grundkörpers in der Malleolenregion an der Außenseite und /oder an der Innenseite des Sprunggelenkes ausgebildet. Damit erstreckt sich die Pelotte sowohl über den lateralen Malleolus als auch über den medialen Malleolus. Es sind jedoch auch Ausführungen von Sprunggelenkbandagen gemäß der Erfindung denkbar, welche je nur eine Pelotte am lateralen Malleolus oder am medialen Malleolus umfassen.

Als ein weiteres, die Erfindung wesentliches verbesserndes Merkmal ist vorgesehen, dass die Sprunggelenkbandage einen Pronationszügel zur Stabilisierung der Pronationsstellung des Fußes umfasst, wobei der Pronationszügel lateral außenseitig an der Bandage angeordnet ist und eine nicht dehnbare Textillage umfasst. Ähnlich wie auch bei Sprunggelenkorthesen wird damit die Pronationsstellung des Fußes nicht nur propriozeptiv, sondern zusätzlich auch mechanisch unterstützt. Dieser Zügel hilft zugleich, den Tallusvorschub zu begrenzen und das vordere äußere Seitenband zu entlasten, was insbesondere durch die lateral außenseitig ausgebildete Anordnung des Zügels unterstützt wird. Der Zügel wird automatisch bei jeder Umknickbewegung und bei einer Streckung des Fußes gespannt, da sich dieser vom lateralen Bereich der Fußsohle beginnend diagonal bis etwa über die Höhe des vorderen Bereichs des Sprungbeins erstreckt. Dazu trägt auch bei, wenn die Textillage A-förmig konturiert ist.

Weil das obere Sprunggelenk in gestreckter Stellung des Fußes besonders labil ist, kann ein leichtes Anheben des äußeren Fußrandes hier eine zusätzliche Sicherheit geben. Die Fußsohle (Planta pedis) weist einen lateralen Rand auf, welche den lateralen Abschluss der Lauffläche bildet. Etwa oberhalb dieses Bereiches beginnt der Pronationszügel, welcher gemäß der A-Form zwei Zügelstränge umfasst, welche bis etwa über die Höhe des vorderen Bereichs des Sprungsbeins zusammenlaufen. Entsprechend der A-Form erstrecken sich zwischen den beiden im Wesentlichen vertikal verlaufenden Hauptzügeln zwei Querzügel, welche diagonal zwischen den beiden im Wesentlichen vertikal angeordneten Hauptzügeln verlaufen.

Der A-förmige Pronationszügel weist bevorzugt eine Höhe von insgesamt etwa 105 - 115 mm auf, wobei sich dieser anatomisch an die Form der Fußaußenwölbung anpasst. Die Breite der vertikal verlaufenden Hauptzügel beträgt etwa 12 - 18 mm, wobei das nicht dehnbare Material des Pronationszügels eine vliesartige Oberfläche aufweist.

Vorteilhafterweise ist der Pronationszügel mittels eines Klebemittels auf der Außenseite des dehnbaren Materials des Grundkörpers aufgeklebt. Damit ist eine sichere Verbindung zwischen dem dehnbaren und dem nicht dehnbaren Material hergestellt, wobei durch die flächenförmige Verbindung durch das Klebemittel die Zugkraft des Pronationszügels gleichmäßig in das dehnbare Textilmaterial des Grundkörpers eingeleitet wird. Ergänzend oder Alternativ besteht die Möglichkeit, dass der Pronationszügel auf der Außenseite des dehnbaren Textilmaterials des Grundkörpers aufgenäht ist. Eine weitere Möglichkeit einer Verbindung zwischen dem Pronationszügel und dem dehnbaren Textilmaterial des Grundkörpers kann in einem Textilschweißverfahren gesehen werden, bei dem die beiden Textilmaterialien aneinander geschweißt werden. Dabei ist auch jede weitere Verbindungsart zwischen dem Material des Pronationszügels und dem Material des Grundkörpers denkbar.

Als eine weitere, die Erfindung wesentlich verbessernde Maßnahme ist vorgesehen, dass die Bandage wenigstens einen Pelottenkörper aufweist, welcher im Bereich bzw. im Verlauf der Sehnen der Peronealgruppe angeordnet ist, und sich L-förmig vom unteren Unterschenkelabschnitt in den Fußabschnitt um die Malleolenregion fersenseitig bzw. fußsohlenseitig herum erstreckt. Für die aktive Sicherung der Pronationsstellung des Fußes sind insbesondere die Muskeln der Peronealgruppe zuständig. Diese Muskeln heben den äußeren Fußrand, stabilisieren das Sprunggelenk und tragen so aktiv dazu bei, die überaus häufigen Inversionstraumen zu vermeiden. Diese Außenpelotte folgt dem Verlauf der Sehnen der Pronatoren und spannt diese vor. Ähnlich wie bei einer Patellasehnenbandage kann dadurch der Tonus der sichernden Muskeln erhöht und ihr Ansprechverhalten positiv beeinflusst werden.

Das obere und das untere Sprunggelenk bilden eine Einheit, und beide Gelenke formen zusammen eine Art Kardangelenk. Dieses erlaubt es, den Fuß in gebeugter oder gestreckter Position (dorsalflektiert oder plantarflektiert) auf unterschiedlich geneigtem Untergrund sicher aufzusetzen. Die Außenpelotte trägt dabei im unteren Sprunggelenk dazu bei, das Calcaneocuboidal-Gelenk, welches ein Teilgelenk des unteren Sprunggelenks bildet, zu stützen und zu stabilisieren. Die Außenpelotte stützt dieses Gelenk auf zweifache Weise: Das Silikonpolster auf der Außenseite des Fußes puffert direkte Gewalteinwirkungen ab und die Aktivierung der Peronealgruppe unterstützt die sichere Funktion des Bandapparates in diesem Teilgelenk. Daher bietet die Ausformung der Außenpelotte unter Einschluss des Calcaneocuboidal- Gelenks eine besonders vorteilhafte Ausbildungsform. Der Pelottenkörper kann dabei als Silikonkissen ausgebildet sein, wobei auch andere gelartige bzw. weichelastische Materialien einsetzbar sind.

Vorteilhafterweise ist der Pelottenkörper in einer medial am Grundkörper ausbildeten Tasche aufgenommen, wobei die Tasche zwischen einer an den Grundkörper angebrachten zweiten Textillage sowie dem Grundkörper selbst gebildet ist. Das heißt, dass das Silikonkissen durch eine Textillage innenseitig auf der Seite des Fußes auf den Grundkörper aufgenäht ist, und umschließt somit das Silikonkissen. Auch hinsichtlich dieses Pelottenkörpers besteht die Möglichkeit, diesen an der Außenseite und /oder an der Innenseite des Sprunggelenkes seitlich anzuordnen. Dabei sind Varianten denkbar, bei denen die Bandage entweder nur außenseitig, nur innenseitig oder auf beiden Seiten ein Silikonkissen umfasst.

Weitere, die Erfindung verbessernde Maßnahmen sind in den Unteransprüchen angegeben oder werden nachfolgend gemeinsam mit der Beschreibung eines bevorzugten Ausführungsbeispiels der Erfindung anhand der beiden Figuren näher dargestellt. Es zeigt:
- Fig.1: eine Ansicht eines Fußes, über welchen eine Sprunggelenkbandage gezogen ist, welche eine Pelotte in der Malleolenregion, einen Pronationszügel sowie einen Pelottenkörper im Verlauf der Sehnen der Peronealgruppe aufweist; und
- Fig.2: zeigt einen Querschnitt durch eine Sprunggelenkbandage gemäß der Schnittebene II - II in Fig.1.

Bei den Figuren handelt es sich lediglich um eine beispielhafte technische Ausführung der vorliegenden Erfindung.

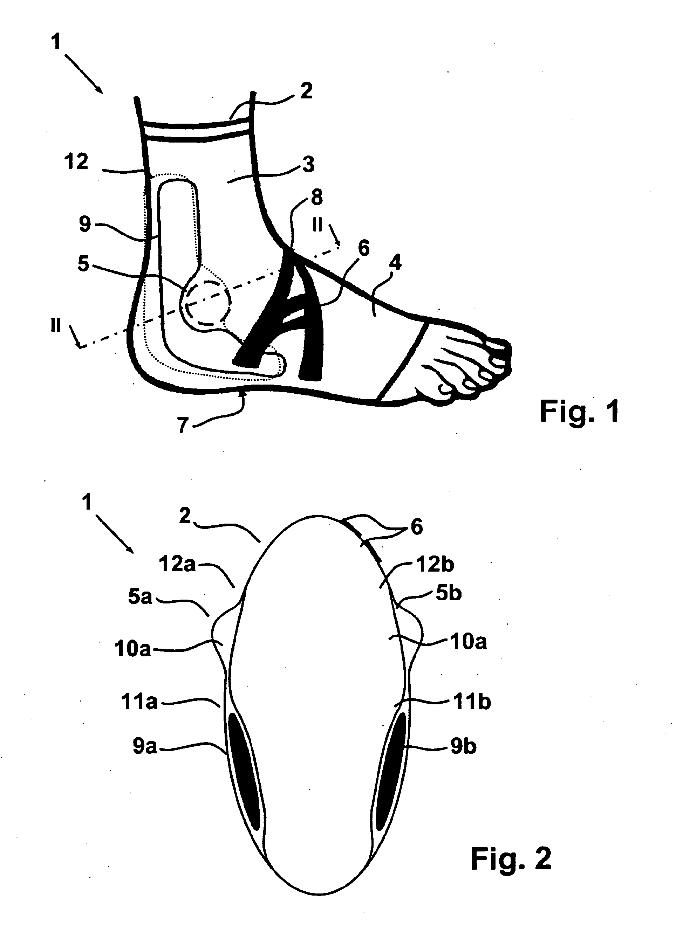

Fig.1 zeigt eine Ansicht einer Sprunggelenkbandage 1, welche über einen rechten Fuß gezogen ist. Die Sprunggelenkbandage 1 ist aus einem socken- oder strumpfartigen Grundkörper 2 gebildet, welcher sich aufgrund seiner Elastizität an die Anatomie des Fußes anpasst. Die Bandage erstreckt sich von einem Unterschenkelabschnitt 3 über die Ferse und das Sprunggelenk in einen Fußabschnitt 4, und ist einteilig ausgeführt. Im Bereich der Malleolenregion ist eine Pelotte 5 schematisch angedeutet, welche als 3D-Gestrick im Grundkörper 2 ausgeformt ist. Um die Malleolenregion herum erstreckt sich ein Pelottenkörper 9, welcher im Bereich bzw. im Verlauf der Sehnen der Peronealgruppe angeordnet ist, und sich L-förmig vom unteren Unterschenkelabschnitt 3 in den Fußabschnitt 4 um die Malleolenregion fersenseitig bzw. fußsohlenseitig herum erstreckt. Der Pelottenkörper 9 ist dabei im Bereich der Pelotte 5 mit einer Aussparung versehen, so dass sich diese beiden Bereiche nicht überschneiden. Somit umschließt zwar der Pelottenkörper 9 die Malleolenregion, jedoch verläuft dieser nicht über den Malleolenknochen.

Der Pelottenkörper 9 ist als Silikonkissen ausgestaltet, welches medial am Grundkörper 2 angeordnet ist, so dass das dehnbare Textilmaterial des Grundkörpers 2 außenseitig eine durchgehende Struktur aufweist. Die Befestigung des Pelottenkörpers 9 am Grundkörper 2 ist mittels einer Textillage realisiert, welche gemäß dem Verlauf der Naht 12 innenseitig am Grundkörper 2 aufgenäht ist. An der lateralen Seite der Bandage ist ein Pronationszügel 6 angebracht, welcher mittels eines Klebeverfahrens auf das Textilmaterial des Grundkörpers 2 aufgeklebt ist. Der Pronationszügel 6 hat eine A-förmig konturierte Grundform, und besteht aus einer Textillage, welcher aus einem nicht dehnbaren, zug- und reißfesten Material ausgebildet ist. Der Pronationszügel 6 erstreckt sich diagonal vom lateralen Bereich der Fußsohle 7 beginnend bis etwa über die Höhe des vorderen Bereiches des Sprungbeines 8. Das Sprungbein 8 - hier nicht sichtbar dargestellt - beschreibt die Region, innerhalb derer etwa der Knoten des Schnürriemens eines Halbschuhs gelegen ist. Gemäß der A-Form treffen dabei zwei Hauptzügel zusammen, wobei die beiden Hauptzügel im Wesentlichen A-förmig in Richtung der Fußsohle auseinander laufen. Zwischen den beiden Hauptzügeln sind zwei kleinere Diagonalzügel integriert, wobei sämtliche Zügel aus einer einteiligen, nicht dehnbaren Textillage hergestellt sind. Die Außenseite der Textillage weist eine fließförmige bzw. filzförmige Oberfläche auf, um eine Reibung beispielsweise in einem Schuh zu minimieren.

Fig.2 zeigt einen Querschnitt gemäß der Schnittebene II - II, welche in Fig.1 angedeutet ist. Hierin ist die Sprunggelenkbandage 1 im Querschnitt dargestellt, so dass der Grundkörper 2 in der für diese Schnittebene resultierenden Kontur dargestellt ist. Entsprechend der Ausformung der Malleolenregion werden die Malleolen mittels einer Pelotte 5 umschlossen, wobei sowohl die laterale Malleole als auch die mediale Malleole mit jeweils einer Pelotte 5a, 5b umschlossen ist. Diese ist als 3D-Gestrick im dehnbaren Textilmaterial des Grundkörpers 2 selbst ausgeformt und umfasst keinen externen Formkörper oder ähnliches. Auf der medialen Seite des Grundkörpers 2 ist sowohl innenseitig als auch außenseitig am Sprunggelenk jeweils eine zweite Textillage 10 mittels einer Naht 12 aufgenäht, um eine innerhalb des Grundkörpers 2 gelegene Tasche 11 auszubilden. Innerhalb dieser Taschen 11a, 11b sind Pelottenkörper 9 eingesetzt, welche als Silikonkissen ausgebildet sind. Diese haben einen linsenförmigen Querschnitt, und unterstützen vor allem die Muskeln der Peronealgruppe. Die Anordnung der als Außenpelotten gebildeten Pelottenkörper 9 folgt dem Verlauf der Sehnen der Pronatoren der Peronealgruppe und spannt diese vor. Somit ist die Formgebung des Pelottenkörpers 9 dem Verlauf der Sehnen der Peronealgruppe angepasst.

Die vorliegende Erfindung beschränkt sich in ihrer Ausführung nicht auf das vorstehend angegebene, bevorzugte Ausführungsbeispiel. Vielmehr ist eine Anzahl von Varianten denkbar, welche von der dargestellten Lösung auch bei grundsätzlich anders gearteten Ausführungen Gebrauch macht.

### Bezugszeichenliste

- **1**: Sprunggelenkbandage
- **2**: Grundkörper
- **3**: Unterschenkelabschnitt
- **4**: Fußabschnitt
- **5**: Pelotte
- **6**: Pronationszügel
- **7**: Fußsohle
- **8**: Sprungbein
- **9**: Pelottenkörper
- **10**: Textillage
- **11**: Tasche
- **12**: Naht

## Patentansprüche

1. Sprunggelenkbandage (1) mit einem anatomisch geformten socken- oder strumpfartigen Grundkörper (2), welcher sich zumindest zwischen einem Unterschenkelabschnitt (3) und einem Fußabschnitt (4) erstreckt und aus einem dehnbaren Textilmaterial ausgebildet ist, wobei die Bandage im Bereich über wenigstens einer Malleolenregion eine Pelotte (5) umfasst, **dadurch gekennzeichnet, dass** die zumindest eine Pelotte (5) als 3D-Gestrick im Grundkörper (2) ausgeformt ist, wobei das dehnbare Textilmaterial des Grundkörpers (2) im Übergang in das die Pelotte (5) bildende 3D-Gestrick eine Texturänderung aufweist, welche eine lateral nach außen gerichtete Dehnbarkeit bewirkt, die höher ist als die Dehnbarkeit des Textilmaterials des Grundkörpers (2).

2. Sprunggelenkbandage nach Anspruch 1, **dadurch gekennzeichnet, dass** das dehnbare Textilmaterial einen gummiartigen Gewebeanteil aufweist.

3. Sprunggelenkbandage nach Anspruch 2, **dadurch gekennzeichnet, dass** die Texturänderung des dehnbaren Textilmaterials im Übergang vom Grundkörper (2) in das 3D-Gestrick eine Reduzierung des gummiartigen Gewebeanteils umfasst.

4. Sprunggelenkbandage nach Anspruch 2 oder 3, **dadurch gekennzeichnet, dass** der gummiartige Gewebeanteil des dehnbaren Textilmaterials eine Vorspannung aufweist.

5. Sprunggelenkbandage nach Anspruch 4, **dadurch gekennzeichnet, dass** die Vorspannung im Bereich des 3D-Gestrickes geringer ist als im dehnbaren Textilmaterial des Grundkörpers (2).

6. Sprunggelenkbandage nach einem Ansprüche 1 bis 5, **dadurch gekennzeichnet, dass** das die Pelotte (5) bildende 3D-Gestrick kreisförmig ausgebildet ist, wobei der Durchmesser des kreisförmigen 3D-Gestrickes im ungedehnten Zustand etwa 15mm bis 35mm, vorzugsweise etwa 20mm bis 30mm und besonders bevorzugt etwa 25mm aufweist.

7. Sprunggelenkbandage nach einem der Ansprüche 1 bis 6, **dadurch gekennzeichnet, dass** das die Pelotte (5) bildende 3D-Gestrick im dehnbaren Textilmaterial des Grundkörpers (2) in der Malleolenregion an der Außenseite und/oder an der Innenseite des Sprunggelenkes ausgebildet ist.

8. Sprunggelenkbandage nach einem der Ansprüche 1 bis 7, **dadurch gekennzeichnet, dass** die Bandage einen Pronationszügel (6) zur Stabilisierung der Pronationsstellung des Fußes umfasst, wobei der Pronationszügel (6) lateral an der Bandage angeordnet ist und eine nicht dehnbare Textillage umfasst.

9. Sprunggelenkbandage nach Anspruch 8, **dadurch gekennzeichnet, dass** die Textillage A-förmig konturiert ist, wobei sich der Pronationszügel (6) vom lateralen Bereich der Fußsohle (7) beginnend diagonal bis etwa über die Höhe des vorderen Bereiches des Sprungbeines (8) erstreckt.

10. Sprunggelenkbandage nach Anspruch 8 oder 9, **dadurch gekennzeichnet, dass** der Pronationszügel (6) mittels eines Klebemittels auf der Außenseite des dehnbaren Textilmaterials des Grundkörpers (2) aufgeklebt ist, dass der Pronationszügel (6) auf der Außenseite des dehnbaren Textilmaterials des Grundkörpers (2) aufgenäht ist oder dass der Pronationszügel (6) mittels eines Textilschweißverfahrens auf der Außenseite des dehnbaren Textilmaterials des Grundkörpers (2) aufgeschweißt ist..

11. Sprunggelenkbandage nach einem der Ansprüche 1 bis 10. **dadurch gekennzeichnet, dass** die Bandage wenigstens einen Pelottenkörper (9) aufweist, welcher im Bereich bzw. im Verlauf der Sehnen der Peronealgruppe angeordnet ist und sich L-förmig vom unteren Unterschenkelabschnitt (3) in den Fußabschnitt (4) um die Malleolenregion fersenseitig bzw. fußsohlenseitig herum erstreckt.

12. Sprunggelenkbandage nach Anspruch 11, **dadurch gekennzeichnet, dass** der Pelottenkörper (9) aus einem Silikonkissen gebildet ist.

13. Sprunggelenkbandage nach Anspruch 11 oder 12, **dadurch gekennzeichnet, dass** der Pelottenkörper (9) in einer medial am Grundkörper (2) ausgebildeten Tasche (11) aufgenommen ist, wobei die Tasche (11) zwischen einer an den Grundkörper (2) angebrachten zweiten Textillage (10) sowie dem Grundkörper (2) selbst gebildet ist.

14. Sprunggelenkbandage nach Anspruch 13, **dadurch gekennzeichnet, dass** die medial am Grundkörper (2) ausgebildete Tasche (11) durch eine entsprechend der Kontur der Tasche (11) verlaufende Naht (12) an den Grundkörper (2) angenäht ist.

15. Sprunggelenkbandage nach einem der Ansprüche 11 bis 14, **dadurch gekennzeichnet, dass** der Pelottenkörper (9) an der Außenseite und /oder an der Innenseite des Sprunggelenkes angeordnet ist.

## Claims

1. Ankle joint bandage (1) with an anatomically formed sock- or stocking-like basic body (2) lying at least between a lower-leg section (3) and a foot section (4) and formed of a stretchable textile material, with the bandage comprising in the area over at least one malleolar region a pressure pad (5), **characterized in that** the at least one pressure pad (5) is formed as 3-dimensional fabric in the basic body (2) and the stretchable textile material of the basic body (2) shows at its transition to the 3-dimensional fabric forming the pressure pad (5) a variation in texture that effects a stretchability directed laterally outwards and higher than the stretchability of the textile material of the basic body (2).

2. Ankle joint bandage according to Claim 1, **characterized in that** the stretchable textile material has a gum-like fabric part.

3. Ankle joint bandage according to Claim 2, **characterized in that** the texture variation of the stretchable textile material comprises at its transition from the basic body (2) to the 3-dimensional fabric a reduced gum-like fabric part.

4. Ankle joint bandage according to Claims 2 or 3, **characterized by** the gum-like fabric part of the stretchable textile material shows a pretension.

5. Ankle joint bandage according to Claim 4, **characterized in that** the pretension in the area of the 3-dimensional fabric is less than in the stretchable textile material of the basic body (2).

6. Ankle joint bandage according to one of the Claims 1 to 5, **characterized in that** the 3-dimensional fabric forming the pressure pad (5) is in form of a circle and the diameter of the circular 3-dimensional fabric in non-stretched condition is about 15mm to 35mm, preferably about 20mm to 30mm and most preferably about 25mm.

7. Ankle joint bandage according to one of the Claims 1 to 7, **characterized in that** the 3-dimenisonl fabric forming the pressure pad (5) and being within the stretchable textile material of the basic body (2) is formed at the outside of the malleolar region and/or the inside of the ankle joint.

8. Ankle joint bandage according to one of the Claims 1 to 7, **characterized in that** the bandage comprises a pronator rein (6) to stabilize the prone position of the foot, with the pronator rein (6) being arranged laterally at the bandage and comprises a non-stretchable textile ply.

9. Ankle joint bandage according to Claim 8, **characterized in that** the textile ply has A-shaped contours and that the pronator rein (6) reaches starting from the lateral region of the sole of the foot (7) diagonally to about above the level of the front region of the anklebone (8).

10. Ankle joint bandage according to Claims 8 or 9, **characterized in that** the pronator rein (6) is by an adhesive stuck on to the outside of the stretchable textile material of the basic body (2), the pronator rein (6) is sewed on to the outside of the stretchable textile material of the basic body (2), or that the pronator rein (6) is by a textile welding procedure welded to the outside of the stretchable textile material of the basic body (2).

11. Ankle joint bandage according to one of the Claims 1 to 10, **characterized in that** the bandage has at least one pressure pad body (9) that is arranged in the region respectively in the course of the tendons of the peroneal group and reaches L-shaped from the lower lower-leg section (3) round the malleolar region on the heel- respectively the sole-side to the foot section (4).

12. Ankle joint bandage according to Claim 11, **characterized by** the pressure pad body (9) being formed of a silicone cushion.

13. Ankle joint bandage according to Claims 11 or 12, **characterized in that** the pressure pad body (9) is received in a pocket (11) formed medially at the basic body (2), with the pocket (11) being formed between a second textile ply (10) fixed to the basic body (2) and the basic body (2) itself.

14. Ankle joint bandage according to Claim 13, **characterized in that** the pocket (11) formed medially at the basic body (2) is sewed to the basic body (2) by a seam (12) following the contours of the pocket (11).

15. Ankle joint bandage according to one of the Claims 11 to 14, **characterized in that** the pressure pad body (9) is arranged at the outside and/or the inside of the ankle joint.

## Revendications

1. Bandage d'articulation tibio-tarsienne (1) avec un corps de base (2) formé anatomiquement de façon de chaussette ou de bas s'étendant du moins entre une section de la jambe inférieure (3) et une section du pied (4) et fait d'un matériau textile extensible, ce bandage comprenant dans la zone couvrant du moins une région malléolaire un coussin compressif (5), **caractérisé en ce que** le du moins un coussin compressif (5) est formé dans le corps de base (2) comme tricotage à trois dimensions et que le matériau textile extensible du corps de base (2) montre à sa transition au tricotage à trois dimensions formant le coussin compressif (5) une variation de texture effectuant une extensibilité latérale vers l'extérieur et plus élevée que l'extensibilité du matériau textile du corps de base (2).

2. Bandage d'articulation tibio-tarsienne selon la Revendication 1, **caractérisé en ce que** le matériau textile extensible montre un part de tissu gommeux.

3. Bandage d'articulation tibio-tarsienne selon la Revendication 2, **caractérisé en ce que** la variation de texture du matériau textile extensible comprend à sa transition du corps de base (2) au tricotage à trois dimensions une réduction du part de tissu gommeux.

4. Bandage d'articulation tibio-tarsienne selon les Revendications 2 ou 3, **caracbérisé en ce que** le part de tissu gommeux du matériau textile montre une prétension.

5. Bandage d'articulation tibio-tarsienne selon la Revendication 4, **caractérisée en ce que** la prétension dans la région du tricotage à trois dimensions est moins que dans le matériau textile extensible du corps de base (2).

6. Bandage d'articulation tibio-tarsienne selon une des Revendications 1 à 5, **caractérisé en ce que** le tricotage à trois dimensions formant le coussin compressif (5) est de forme circulaire et le diamètre du tricotage à trois dimensions circulaire en état non étendu est de 15mm à 35mm, de préférence environ de 20mm à 30mm, et plus préférentiellement à 25mm environ.

7. Bandage d'articulation tibio-tarsienne selon une des Revendications 1 à 6, **caractérisé en ce que** le tricotage à trois dimensions formant le coussin compressif (5) dans le matériau textile extensible du corps de base (2) est formé dans la région malléolaire à la face extérieure et/ou à la face intérieure de l'articulation tibio-tarsienne.

8. Bandage d'articulation tibio-tarsienne selon une des Revendications 1 à 7, **caractérisé en ce que** le bandage comprend une bride de pronation (6) pour stabiliser la position pronatrice du pied, cette bride de pronation (6) étant arrangée latéralement au bandage et comprenant une couche textile non-extensible.

9. Bandage d'articulation tibio-tarsienne selon la Revendication 8, **caractérisé en ce que** la couche textile a des contours en forme d'A et la bride de pronation s'étend commençant à la région latérale de la plante du pied (7) diagonalement à peu près jusqu'au niveau de la région de devant de l'astragale (8).

10. Bandage d'articulation tibio-tarsienne selon les Revendications 8 ou 9, **caractérisé en ce que** la bride de pronation (6) est par un adhésif collée sur la face extérieure du matériau textile extensible du corps de base (2), la bride de pronation (6) est coudée sur la face extérieure du matériau textile extensible du corps de base (2), ou que la bride de pronation (6) est par une méthode de soudage textile soudée sur la face extérieure du matériau textile extensible du corps de base (2).

11. Bandage d'articulation tibio-tarsienne selon une des Revendications 1 à 10, **caractérisé en ce que** le bandage montre du moins un corps de coussin compressif (9) qui est arrangé dans la région respectivement le long des tendons du groupe péronier et s'étend en forme d'L de la section basse de la jambe inférieure (3) autour de la région malléolaire au côté du talon respectivement de la plante du pied à la section du pied (4).

12. Bandage d'articulation tibio-tarsienne selon la Revendication 11, **caractérisé en c e que** le corps du coussin compressif (9) est formé d'un coussin de silicone.

13. Bandage d'articulation tibio-tarsienne selon les Revendications 11 ou 12, **caractérisé en ce que** le corps du coussin compressif (9) est logé dans une poche (11) formée médianement au corps de base (2), laquelle poche (11) étant formée entre une deuxième couche textile (10) attachée au corps de base (2) et le corps de base (29 même.

14. Bandage d'articulation tibio-tarsienne selon la Revendication 13, **caractérisé en ce que** la poche (11) formée médianement au corps de base 82) est coudée au corps de base (2) par une couture (12) qui suit les contours de la poche (11).

15. Bandage d'articulation tibio-tarsienne selon une des Revendications 11 à 14, **caractérisé en ce que** le corps du coussin compressif (9) est arrangé à la face extérieure et/ou à la face intérieure de l'articulation tibio-tarsienne
